# EUROPEAN PATENT SPECIFICATION

(11) **EP 2 430 076 B1**
(45) Date of publication and mention of the grant of the patent: **11.06.2014**
(21) Application number: 10766534.1
(22) Date of filing: 07.04.2010
(51) Int. Cl.: C08H 8/00, C08B 1/00, C13K 1/02, C12P 7/10, C12P 19/14, D21B 1/36

(54) **PRETREATMENT OF LIGNOCELLULOSIC BIOMASS THROUGH REMOVAL OF INHIBITORY COMPOUNDS**
VORBEHANDLUNG VON LIGNOZELLULOSEHALTIGER BIOMASSE DURCH ENTFERNUNG VON HEMMERVERBINDUNGEN
PRÉTRAITEMENT DE BIOMASSE LIGNOCELLULOSIQUE PAR ÉLIMINATION DE COMPOSÉS INHIBITEURS

(30) Priority: 20.04.2009 US 170805 P
(43) Date of publication of application: 21.03.2012
(73) Proprietor: Greenfield Ethanol Inc., Toronto, Ontario M5C 2B8 (CA)
(72) Inventor: DOTTORI, Frank, A., Temiscaming Québec J0Z 3R0 (CA); BENSON , Robert , Ashley Cooper, North Bay Ontario P1A 3Y1 (CA); BENECH, Régis-Olivier, Chatham Ontario N7M 5J5 (CA)
(74) Representative: Tombling, Adrian George
(86) International application number: PCT/CA2010/000483
(87) International publication number: WO 2010/121348

(56) References cited:
- WO-A2-2009/108773
- CA-A1- 2 615 844
- CA-B- 1 217 765
- DE-A1- 19 730 486
- US-A- 4 908 099
- US-A1- 2004 016 525
- US-A1- 2008 057 555
- US-A1- 2010 065 128

## Description

### FIELD OF THE INVENTION

The present invention generally relates to the production of ethanol from biomass and in particular to a process for the pretreatment of lignocellulosic biomass, like it is known from US 2008/0057555 A1.

### BACKGROUND OF THE INVENTION

World energy consumption is predicted to increase 54 % between 2001 and 2025. Considerable research effort is being directed towards the development of sustainable and carbon neutral energy sources to meet future needs.

Biofuels are an attractive alternative to current petroleum-based fuels, as they can be utilized in transportation with little change to current technologies and have significant potential to improve sustainability and reduce greenhouse gas emissions.

Biofuels include fuel ethanol. Fuel ethanol is produced from biomass by converting starch or cellulose to sugars, fermenting the sugars to ethanol, and then distilling and dehydrating the ethanol to create a high-octane fuel that can substitute in whole or in part for gasoline.

In North America, the feedstock for the production of fuel ethanol is primarily corn, while in Brazil sugar cane is used. There are disadvantages to using potential food or feed plants to produce fuel. Moreover, the availability of such feedstocks is limited by the overall available area of suitable agricultural land. Therefore, efforts are being made to generate ethanol from non-food sources, such as cellulose, and from crops that do not require prime agricultural land.

One such non-food source is lignocellulosic biomass. Lignocellulosic biomass may be classified into four main categories: (1) wood residues (sawdust, bark or other), (2) municipal paper waste, (3) agricultural residues (including corn stover, corncobs and sugarcane bagasse), and (4) dedicated energy crops (which are mostly composed of fast growing tall, woody grasses such as switchgrass and miscanthus).

Lignocellulosic biomass is composed of three primary polymers that make up plant cell walls: Cellulose, hemicellulose, and lignin. Cellulose fibres are locked into a rigid structure of hemicellulose and lignin. Lignin and hemicelluloses form chemically linked complexes that bind water soluble hemicelluloses into a three dimensional array, cemented together by lignin. Lignin covers the cellulose microfibrils and protects them from enzymatic and chemical degradation. These polymers provide plant cell walls with strength and resistance to degradation, which makes lignocellulosic biomass a challenge to use as substrate for biofuel production.

There are two main approaches to the production of fuel ethanol from biomass: thermochemical and biochemical. Thermochemical processes convert the biomass to a reactive gas called syngas. Syngas is converted at high temperature and pressure to ethanol by a series of catalyzed processes. Biochemical processes use biocatalysts called enzymes to convert the cellulose and hemicellulose content to sugars, which are then fermented to ethanol and other fuels such as butanol.

Biochemical conversion of lignocellulosic biomass to ethanol in general involves five basic steps (1) Feed preparation - the target biomass is cleaned and adjusted for size and moisture content; (2) Pretreatment - exposure of the raw biomass to high pressure and temperature for a specified duration; with or without catalyzing additives; (3) Hydrolysis - conversion of the pretreated biomass to simple sugars using special enzyme preparations to hydrolyze pretreated plant cell-wall polysaccharides to a mixture of simple sugars; (4) Fermentation, mediated by bacteria or yeast, to convert these sugars to fuel such as ethanol; and (5) Distillation and Dehydration of the ethanol/fuel.

Pretreatment processes, such as steam explosion, generally result in extensive hemicellulose breakdown and, to a certain extent, to the degradation of xylose and glucose to unwanted byproducts. Certain pretreatment methods may also employ added acids to catalyze the hydrolysis of hemicellulose. Additives such as sulfuric acid are often used when the biomass has insufficient acetate residues on the hemicellulose sugars to acidify the mixture sufficiently. Alkalis may also be added during pretreatment to remove lignin. However, acids and alkalis are themselves inhibitors of hydrolysis and fermentation. Moreover, lignin and some soluble lignin derivatives are toxic to yeast and also have been found to inhibit hydrolysis. Also, the hemicellulose of some feed stocks is highly acetylated which means that the breakdown and liquefaction of the hemicellulose, which occurs during pretreatment, leads to the formation of acetic acid. Acetic acid is a powerful inhibitor of both hydrolysis and fermentation. Hemicellulose decomposition products such as acetic acid, formic acid, furfural and hydroxyl methyl furfural etc., which are produced during biomass pretreatment, remain in the pretreated biomass and carry through to the hydrolysis and fermentation steps, negatively affecting the enzymatic processes and the fermentation process.

A key inhibitor of the catalytic activities of cellulolytic enzymes is the soluble forms of hemicellulose, predominantly the soluble xylo-oligosaccharides, the soluble polymeric chains of xylose. Hemicellulose decomposition products which remain in the pretreated biomass and carry through to the hydrolysis and fermentation steps can negatively affect enzymatic conversion of cellulose to glucose, most predominantly the xylo-oligosaccharides which must be removed. The hemicellulose decomposition products reduce the effectiveness of the cellulose hydrolyzing enzymes, requiring increased levels of added enzyme, the cost of which is an important factor in providing a cost effective commercial process. In addition, the pre-treatment and preparation steps also have a significant impact and the recovery of a usable hemicellulose (etc.) stream for value added use is equally important.

Furthermore, all forms of lignocellulosic biomass have some level of sterols, fatty acids, ethers and other extractives that can also be inhibitory.

Diverse techniques have been explored and described for the pretreatment of size-reduced biomass material with the aim of producing a substrate that can be more rapidly and efficiently hydrolyzed to yield mixtures of fermentable sugars.
These approaches have in common the use of conditions and procedures which greatly increase the surface area to which aqueous reactants and enzymes have access. In particular, they increase the percentage of the cellulose that is opened up to enzymatic hydrolysis of cellulose to glucose. Unfortunately, many of the degradation products released in the pretreatment step are also inhibitors, as described previously, that retard the downstream hydrolysis and fermentation processes. This results in increased capital equipment costs and results in an uneconomical process.

One approach to address the inhibitory effect of all of these substances is the use of harsher pre-treatment conditions, which can for example be tailored to effectively hydrolyze and degrade the hemicellulose to such an extent that very little xylose and xylan oligosaccharides remain to interfere with the cellulose enzymes. However this approach creates another significant disadvantage in that it causes significant cellulose degradation, which then reduces glucose yield and ultimately the ethanol yield, often creating a commercially significant reduction of the overall ethanol process efficiency.

In another approach xylanases are used to completely hydrolyze the xylan oligomers to xylose and lessen the inhibitory effect of these oligomers. However, although this approach is somewhat effective, it produces high levels of xylose which is itself an inhibitor. Moreover, the other inhibitory compounds generated in the pretreatment step are still present. Thus, although the overall yield is better, in the end this approach is not commercially viable due to the added cost for the xylanases and still higher cellulase cost.

Yet a further approach to improving the overall yield is to fully wash the pretreated biomass for removal of all inhibitory compounds. Although this results in improved downstream hydrolysis and fermentation yields, washing of the pretreated biomass to remove all of the inhibitory compounds, which of course would theoretically lead to the best hydrolysis and fermentation yields, is as uneconomical as it is capital intensive. Moreover, this approach is very environmentally unfriendly due to the need for vast quantities of washing fluid, generally water. In addition, this complete removal produces a huge volume of eluent stream that needs to be concentrated at great cost if the eluted compounds are to be disposed of or prepared for other purposes and the eluent recovered for reuse. As with other approaches discussed above, the cost for operating the washing process with the aim to completely remove all inhibitors virtually negates or even exceeds the value of the lower enzyme dosages, reduced processing times, or potentially higher ethanol yield achievable.

In known pretreatment processes in which the inhibitory compounds are not removed prior to hydrolysis the prehydrolysed biomass must be diluted in order to reduce the concentrations of toxic and inhibitory compounds to an acceptable level with respect to cellulolytic enzymes and fermenting organisms. As a result, large amounts of water are required prior to the enzymatic hydrolysis step. This results, not only in increased capital equipment cost (tankage) but also in increased operating cost (fuel) associated with low ethanol yield. High amounts of steam energy are then needed to concentrate the dilute ethanol to the finished product concentration.

Thus, compared to the prior art processes, a more economical and effective approach for dealing with the inhibitor compounds produced during pretreatment is desirable.

### SUMMARY OF THE INVENTION

It is now an object of the present invention to provide a process which overcomes at least one of the above disadvantages by reducing the inhibition impact on the rate of hydrolysis and fermentation of pretreated biomass by degradation and hydrolysis products and other inhibitory compounds produced during pretreatment of lignocellulosic biomass.

It is a further object of the invention to provide a lignocellulosic biomass pretreatment process wherein hemicellulose, hemicellulose degradation and hydrolysis products, cellulose degradation products and other inhibitory compounds typically present in biomass such as fatty acids, sterols, esters, ethers etc. are removed in a commercially viable, economical manner prior to the enzymatic hydrolysis step to achieve the most economical maximization of hydrolysis and fermentation yields.

As is apparent from the above discussion, known approaches to improve the overall ethanol yield by successfully reducing the amount of inhibitory compounds in the pretreated biomass are generally linked to increased cost for operating the respective method. As a result, increased yields are only obtainable at significantly increased costs which are higher overall than the value of the increased ethanol yield or decreased hydrolysis or fermentation times and reduced enzyme costs, rendering existing methods economically unacceptable.

The inventors of the present application have now surprisingly discovered that complete removal of the inhibitory compounds is neither required nor desirable for the achievement of the most economically viable pretreatment process. The inventors have discovered a narrow range of extraction and inhibitory compounds removal conditions at which hemicelluloses and hemicellulose hydrolysis and degradation products and other inhibitors are still present, but reduced to a level where they have a much reduced inhibitory effect on the enzymes. The extraction is achieved with the use of a lower volume of diluent and level of dilution and at equipment cost which requires sufficiently lower additional extraction and compound removal cost to render the process much more cost effective, practical and commercially viable. In effect, the additional extraction cost is thereby significantly less than the value of any increased ethanol yield, enzyme cost reduction or reduced processing time achieved.

The removal of inhibitory compounds can be carried out through many different methods, typically a combination of mechanical pressing and draining, aqueous extraction, solvent extraction, filtering, centrifuging, venting, purging, draining, or the like, with or without the addition of eluents. These removal steps can occur during and/or after the pretreatment process. The removal of inhibitory compounds improves the economics of the process by reducing enzyme load and improving enzyme efficiency and fermentation performance. The term washing used throughout this specification defines removal of inhibitory compounds using water as the eluent.

In another aspect, the inventors have discovered that the xylose oligosaccharide content of the pretreated biomass is the single most determinative factor of hydrolysis inhibition and that operating the process for removing any inhibitory compounds most efficiently can be achieved by simply controlling the xylose content in the treated biomass. The term xylose within this specification includes xylose and xylose-oligosacharides. The term washing used in this specification describes removal of inhibitory compounds using water or other eluents for the inhibitory compounds removal.

### BRIEF DESCRIPTION OF THE DRAWINGS

Other objects and advantages of the invention will become apparent upon reading the detailed description and upon referring to the drawings in which:

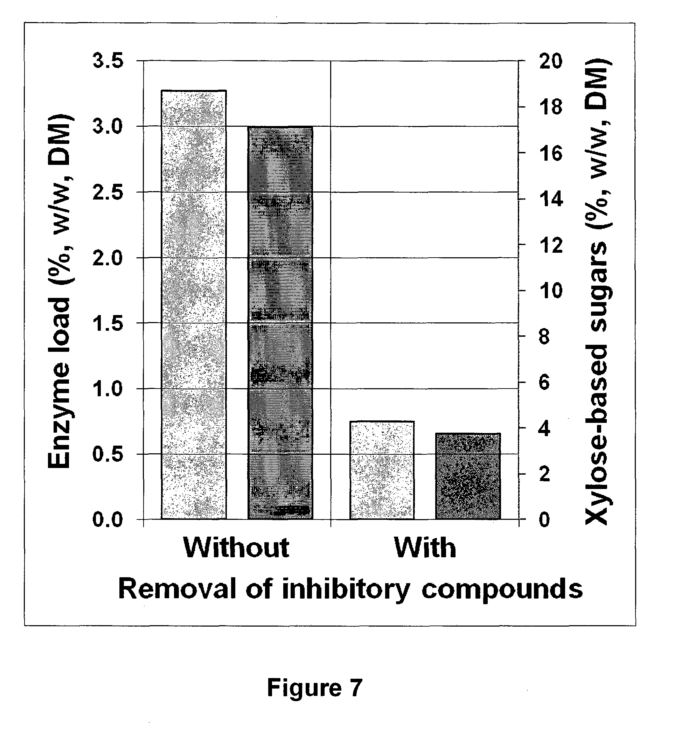

Figure 1A shows the impact of xylose removal by water washing on pretreated corncobs hydrolysis time, i.e. the time to reach 90% of the maximum theoretical cellulose to glucose conversion (t90%, hours). Similar results were obtained with batch and continuous pretreatment. Xylose and Xylo-oligosaccharides content is expressed as percentage dry matter (dm) of xylose. Hydrolysis experiments were carried out at 10 % consistency, a 1 % load of enzyme, 50°C, and pH 5.0. The effect of inhibitor removal on hydrolysis time is even more pronounced at 17% consistency, as seen in Figure 7.
Figure 1B shows the hydrolysis time (t90%) of unwashed and washed pretreated corncobs. Hydrolysis experiments were carried out at 10 % consistency, a 1 % load of enzyme, 50 °C, and pH 5.0.
Figure 2A shows the xylo-oligosaccharides content of unwashed and washed pretreated fibres of corncobs on a dry matter basis.
Figure 2B shows the acetic acid concentration of 17 % consistency corncob slurry produced using unwashed or washed pretreated corncobs.
Figure 3 shows the fermentation time of 17 % corncob hydrolysates unwashed (dashed line) or washed (plain line) prior to enzymatic hydrolysis. Fermentation experiments were carried out at 17 % consistency, 35 °C, pH 5.3 using an industrial grade C6-fermenting yeast, following hydrolysis with a 0.5 % load of enzyme, at 50 °C, a pH 5.0, and at 17 % consistency hydrolysis.
Figure 4A shows a process diagram of the pilot scale (i.e. one metric tonne per day) pretreatment unit used.
Figure 4B shows the process as in Figure 4a where a more practical industrial setup is shown with the washing occurring under pressure prior to pressure release.

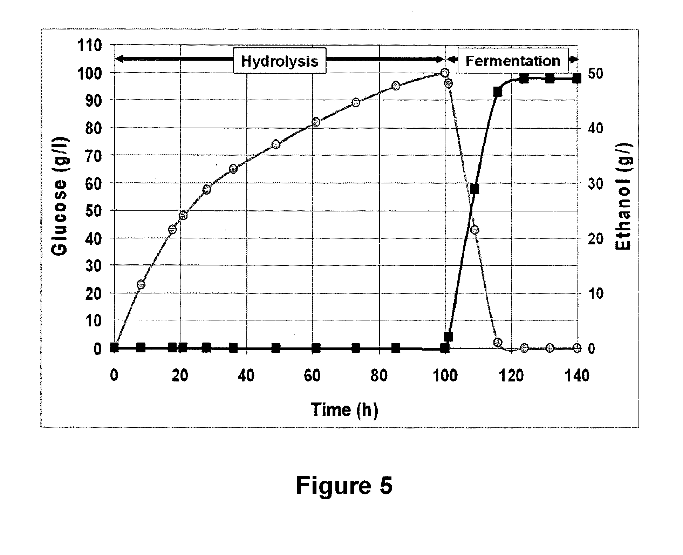

Figure 5 shows hydrolysis and fermentation results of washed pretreated corncobs at pilot scale (2.5 metric tonnes, 17 % consistency). Hydrolysis was carried out at 50°C, and pH 5.0, using a 0.5 % enzyme load. Fermentation was carried out at 33°C, at a pH of 5.3 using industrial grade C6-fermenting yeast. Hydrolysis and fermentation pH adjustment was carried out using liquid ammonia (30 %). Grey circles indicate the glucose concentration. Black squares indicate the ethanol concentration.
Figure 6 illustrates the impact of wash- ratio (single stage washing) on corncobs pre-hydrolysate content of xylo-oligomers and resulting t90% values of 10 % consistency hydrolysis. The xylose based sugars content plotted on the x-axis represents xylan and xylan hydrolysis monomers and oligomers (Xylo-oligosaccharides).
Figure 7 illustrates the impact of inhibitory compounds removal on corncobs pre-hydrolysate content of xylose-based sugars (xylose and xylo-oligomers) (light grey columns) and resulting enzyme load (dark grey columns) required to reach 90% of the maximum theoretical cellulose to glucose conversion by 100 hours hydrolysis of 17 % consistency corn cobs hydrolysate.
Figure 8 shows the relationship between the amount of washing water needed for the achievement of a specific xylose dry matter content in the pretreated biomass when a commercial 2-stage counter current washing process is used.

### DETAILED DESCRIPTION OF PREFERRED EMBODIMENTS

Before explaining the present invention in detail, it is to be understood that the invention is not limited to the preferred embodiments contained herein. The invention is capable of other embodiments and of being practiced or carried out in a variety of ways.

It is to be understood that the phraseology and terminology employed herein are for the purpose of description and not of limitation.

The abbreviations used in the Figures have the following meaning:
°C, temperature in degree Celsius
ms, millisecond
DM, Dry matter
t _{90 %}, time (hours) to reach 90 % of the maximum theoretical conversion of cellulose to glucose.

The invention is directed to lignocellulose pretreatment processes that condition biomass for biochemical conversion into biofuels. These processes produce inhibitors to the downstream biochemical process. The invention reduces these inhibitors by removing them from the biomass, thus improving the process. These inhibitors consist of hemicellulose, hemicellulose hydrolysis and degradation products, cellulose degradation and other inhibitory compounds typically present in biomass such as fatty acids, sterols, esters, ethers etc. These compounds negatively affect the enzymatic hydrolysis and subsequent fermentation processes which are critical to the economics of the process.

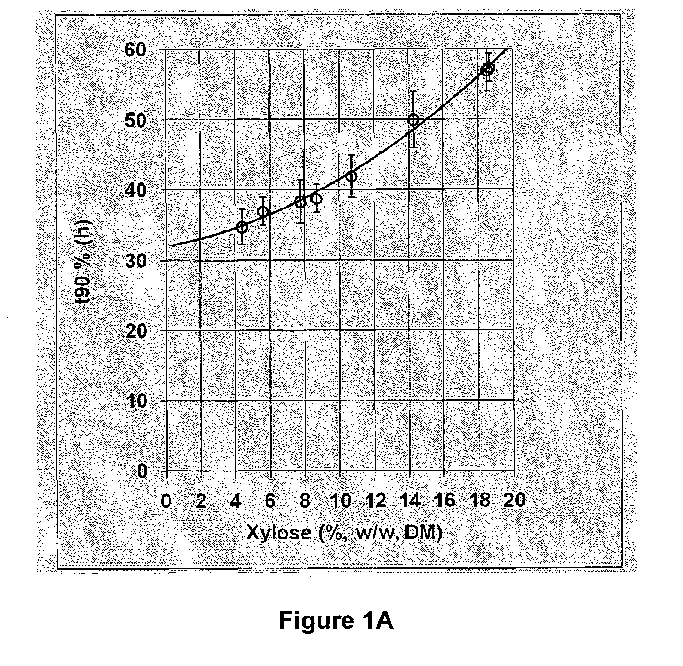

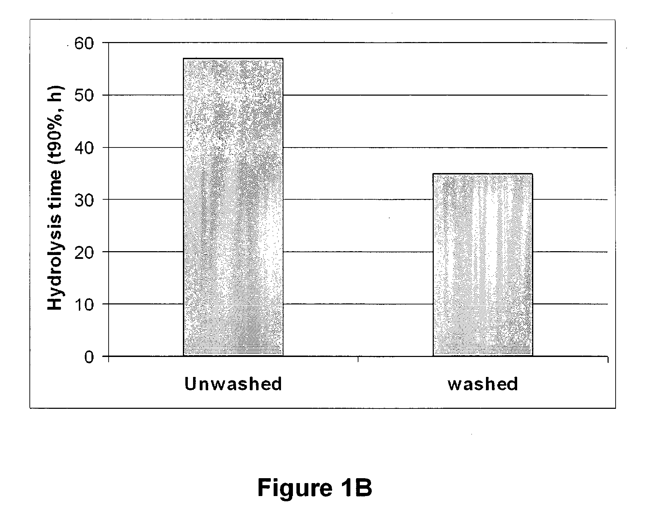

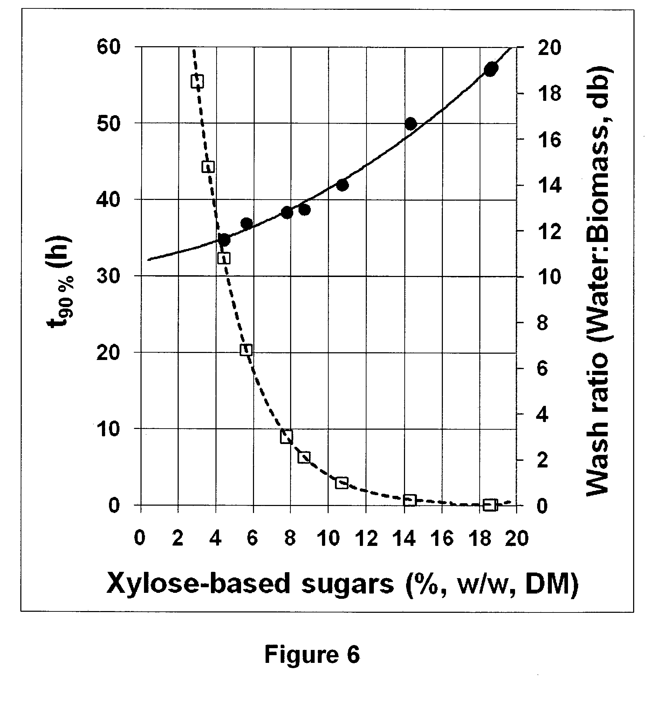

In an exemplary pretreatment process for corn cobs, for example, it was shown that removing 80% to 90% of the hemicellulose and hemi hydrolysis and degradation stream is effective and still commercially viable. As seen in Figure 1, a clear correlation exists between xylose (xylose and Xylo-oligosaccharides) content and cellulose to glucose conversion. The Figure also illustrates that the added incremental yield obtained by reducing the xylose content progressively decreases below about 8% of xylose (w/w dry matter) and becomes small at xylose dm contents below 4%. Furthermore, Figure 6 shows that the diluent amount needed for xylose removal increases exponentially with each additional percent of dry matter extracted below a xylose dry matter content of 10%.

In general, the removal of inhibitory compounds applies to all lignocellulosic biomass feedstock such as bagasse, grass and wood and can be described as a ratio of cellulose to hemicellulose (as hydrolysis and degradation products) remaining post pre-treatment and extracting steps. Theoretically, one would expect to see an increase in enzymatic activity with an increase in this ratio, with the theoretically highest possible ratio attainable at a hemicellulose content of 0%. However, the inventors of the present invention have now, surprisingly, discovered that the ratio of remaining hemicellulose hydrolysis products to cellulose is of little consequence to the enzymatic activity. The inventors further discovered that it is the actual amount of dry matter (dm) of hemicellulose hydrolysis products, in particular xylose oligosaccharides, in the remaining cellulose prehydrolysate which is determinative of the enzyme activity.

The inventors have found that a dm content of xylose (xylose and Xylo-oligosaccharides) from 3% to 10 % is preferred. This is much higher than the 0% content theoretically expected. The most effective level is between 4% and 9% and, since the benefit below 6% in terms of potentially increased ethanol yield, reduced enzyme costs or processing time is counteracted by the exponentially increasing added cost of extraction, for example, in terms of eluent used and the cost for downstream eluent disposal or recovery, a xylose dry matter content of 6% is preferred.

The inhibitory compounds are removed through many different methods, for example by mechanical compressing and draining, aqueous extraction and/or solvent extraction, filtering, centrifuging, pressing, venting, draining, or purging and the like with or without the addition of eluents. These removal steps can occur during and/or after the pretreatment process.

The removed inhibitors are collected and concentrated for value added applications. Efficient collection and cost effective use and value addition of these inhibitors is further beneficial to the economic viability.

In one embodiment, inhibitors are removed during pretreatment by venting volatiles with strategically placed vents to cyclones installed throughout the pressurized pretreatment apparatus and stages.

In another embodiment, inhibitors are removed during pressurized pretreatment by draining or purging liquefied inhibitors. This can be accomplished for example with a simple drain at the lower portion of one of the vessels where condensed liquid collects, or with a complex mechanical apparatus called a screw drainer. The inhibitors, containing liquid, drains out of the biomass without the aid of directed or deliberate mechanical compression; it drains on its own via gravity.

In another embodiment, inhibitors are removed during pressurized pretreatment by draining or purging with the addition of a single or sequential addition of some type of eluent, typically water. The eluent is mixed with the biomass and carries away inhibitors via gravity and is removed to recover eluent consisting of the eluent and soluble solids. This is accomplished in a continuous pretreatment apparatus with a drainer screw. In a screw drainer a mechanical screw transports the biomass upward at a steep angle. Water is added near the top and allowed to filter down through the material and to exit through the screen, pooling at the bottom for collection. The addition of the eluent allows for a greater reduction in the amount of inhibitors extracted. The level of inhibitors can be further reduced by repeating the process in series until desired levels are achieved.

In yet another embodiment the use of an eluent in the removing step can be executed in a counter current washing method.

It is understood by those skilled in the art that the use of eluent will enhance the ability of all liquid removing methods to reduce inhibitors. Those skilled in the art will also understand that it is important to have an inhibitor extract that is as concentrated as possible to afford economically effective downstream processing. Thus minimizing the level of eluent is important. If the eluent is water this could be described as aqueous extraction. If the eluent is alcohol this could be described as organic solvent extraction.

For the purpose of clarity, the liquid extracted from the biomass during and/or just after pretreatment extracted with or without additional eluent can be described in several terms such as "wash water" "inhibitor extract" "xylo-oligosaccharide rich extract", "hemicellulose rich extract", "C5 stream" and the like. Although the term "washing" is often used to describe an aqueous eluent aided removal step, other removal methods and eluents are encompassed by this term as discussed herein.

In another embodiment, inhibitors are removed during pressurized pretreatment with the use of mechanical compression or by squeezing the biomass against a screen or drain of some type that allows the biomass to be pressurized and the inhibitor-rich liquid to be released. These are typically accomplished with powerful finely engineered machines such as modular screw devices. These devices are sealed and can run under the heat and pressure conditions of pretreatment. These mechanical compression steps can be repeated in series to increase removal. These mechanical compression steps can be used with an eluent added to further increase the level of removal.

In a further embodiment, inhibitors are removed after pressurized pretreatment with the use of mechanical compression or squeezing against a screen or drain of some types that allows the biomass to build pressure against a screen and the inhibitor rich liquid entrained to be released through the screen and removed. This is typically accomplished with machines such as screw presses and belt presses etc. These mechanical compression steps can be repeated in series to increase overall removal. These mechanical compression steps can be used with an eluent added to further increase the level of removal.

In yet another embodiment, inhibitors are removed after pressurized pretreatment with, for example, the use of batch operated filter presses that pump the treated biomass against a filter, building up a cake that is low in inhibitors. The pumping is then stopped and the cake is collected. This filtering step can be repeated in series to increase removal. These filters can be used with added eluent to further increase the level of removal.

In still another embodiment, it would be common to see draining of impurities followed by compression, and then draining with or without eluent still under pressure during pretreatment, in turn combined with a post pretreatment extraction step via draining and/or filtering in a filter press depending on the pretreatment process and biomass.

In a particular embodiment and illustrative example corn cobs are cleaned, sized and adjusted for moisture to 40-60%. They are then pretreated with steam in a steam gun at temperatures of 152 °C to 226°C (severity index 3.8-4.2) for periods of 3 - 180 min during which time the volatiles are vented and the liquid drained. The condensate is collected at the bottom of the reaction vessel and removed through a drainage valve. The solids expelled from the reaction vessel upon pressure release, also referred to as pre-hydrolysate, are separated from the gaseous reaction products in a cyclone separator, and collected at the bottom of the separator.

Water as eluent is added to the biomass which is then fed to a press that removes most of the liquid reducing the hemicellulose content as xylose to about 6% DM at which point the cellulose is described as being adequately cleaned of inhibitor and transported to the enzymatic hydrolysis step. The liquid removed from the eluent and pretreated biomass can be described as the wash liquid stream.

The remaining cob solids is then reacted with 0.6% enzymes, hydrolyzing greater than 90% of the cellulose to glucose in less than 100 hrs.

Composition analysis was carried out at the analytical laboratory of Paprican (Montreal, Canada), using the TAPPI methods T249 cm-85 and Dairy one (wet chemistry analysis).

Quantification of soluble products from pretreatment, post washing and enzymatic hydrolysis was carried out by HPLC analysis. The target molecules were sugar monomers such as glucose, xylose, xylo-oligosacharides (as xylose) as well as toxic compounds such as different carboxylic acids, namely acetic acid, formic acid, succinic acid and lactic acid and degradation products of carbohydrates such as hydroxyl-methyl-furfural (HMF) and furfural.

The wash liquid stream contained xylo-oligosaccharides, xylose, acetic acid, formic acid, furfural, arabinose, glucose, mannose, galactose and other inhibitory compounds and toxic compounds that affect the hydrolysis and fermentation processes.

The analytical method used to measure xylan, xylo-oligosaccharide and xylose first hydrolyses the sample fully into xylose. This does not provide the ratio of xylose to xylo-oligosaccharides. A modified method was used to determine the extent to which the xylan has been converted to monomers verses oligomers of xylose. It was found that 40-80% of the xylose was present as xylo-oligosacharides after pretreatment.

Figure 1 shows that decreasing the xylo-oligosaccharides (measured as xylose) content by washing decreased the amount of time needed to achieve cellulose to glucose conversion, with the fastest conversion achieved at complete xylose removal. However, the inventors surprisingly found that a complete removal of the xylose is neither required nor desirable for the achievement of the most economically viable pretreatment process.

Through their diligent investigation, the inventors have discovered a narrow range of conditions for extraction and inhibitory compounds removal at which hemicelluloses and hemicellulose hydrolysis and degradation products and other inhibitors are still present, but reduced to a level where they have a much reduced inhibitory effect on the enzymes. The inventors have discovered, that the most preferable and commercially viable extraction process was achieved with the use of a lower than theoretically required volume of diluent and with termination of the extraction at a higher than theoretically optimal level of xylose content. As a result, the extraction was carried out at a level of dilution and at equipment cost which resulted in sufficiently lowered additional extraction and compound removal cost than the theoretically optimal xylose extraction process, thereby rendering the inventive process much more cost effective, practical and commercially viable. As a result of operating the extraction process at less than theoretically optimal extraction levels, the additional cost for carrying out the xylose extraction step in accordance with the invention over and above regular biomass pretreatment becomes significantly less than the value of any increased ethanol yield, lower enzyme dosages, or reduced processing times achieved. This is surprising and contrary to the cost situation associated with extraction to theoretically optimal levels, the complete removal of all xylose, wherein the additional cost for carrying out the xylose extraction step would exceed the value of any increased ethanol yield, lower enzyme dosages, or reduced processing times, as discussed above.

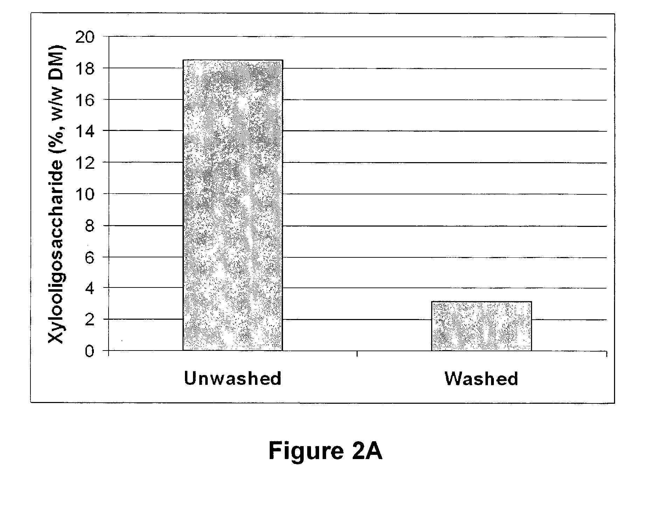

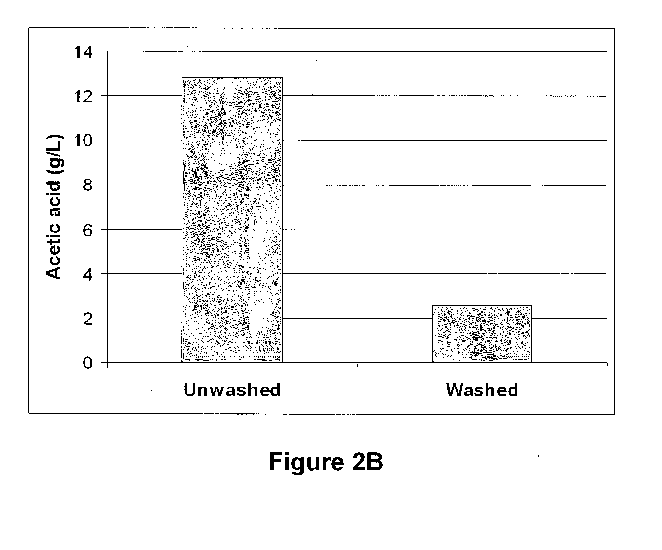

Washing of pretreated biomass is intended to remove impurities. These impurities have a severe impact on the hydrolysis time and the degree of conversion of cellulose to glucose (Figure 1 B). Figures 2A and 2B show the impurities before and after washing of the steam pretreated prehydrolysate.

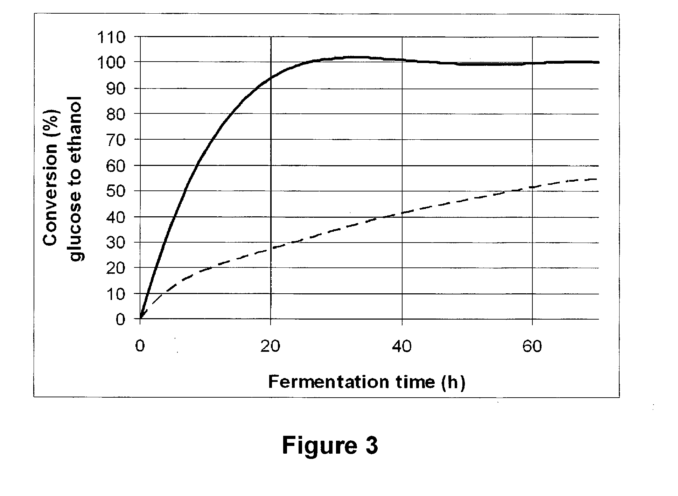

Impurities also increase fermentation time and reduce yield (Figure 3). We have found that xylose (xylose and xylo-oligosaccharides) concentration should be about 6 % w/w overall in the wet washed cobs to minimize hydrolysis time. Acetic acid and other fermentation inhibitors must also be removed in order to minimize fermentation time.

A balance must be maintained between the removal of impurities and the need to minimize the amount of wash water added. Wash water must be concentrated for its eventual re-use. This requires equipment and energy, both of which must be minimized. There are two basic mechanisms for removing impurities by washing- displacement and diffusion. In displacement washing, the impurities are displaced by the washing liquid. In diffusion washing, impurities diffuse from the fibres into the washing liquid. In most practical washing applications both mechanisms play a key role.

Rydholm et al. (1965) refer to two key parameters in the washing process. In the case where the impurities have value such as in Kraft pulping, the recovery of solids is measured as a percentage of the total impurities. If recovery is 100 %, all the solids have been recovered (or all impurities have been removed). The second parameter is the dilution factor. This is usually expressed as tons of water per ton of dry substance. This should be kept as low as possible.

A simple form of washing was used throughout our examples. Biomass at about 35 % DM after pretreatment was diluted with water at to afford a ratio of about 16:1 (water:dm). The diluted biomass was then squeezed in a hydraulic press to bring the consistency up to about 40 % (removal step). The solids were then shredded and diluted to the desired consistency for hydrolysis and fermentation. The recovery factor was >99%.

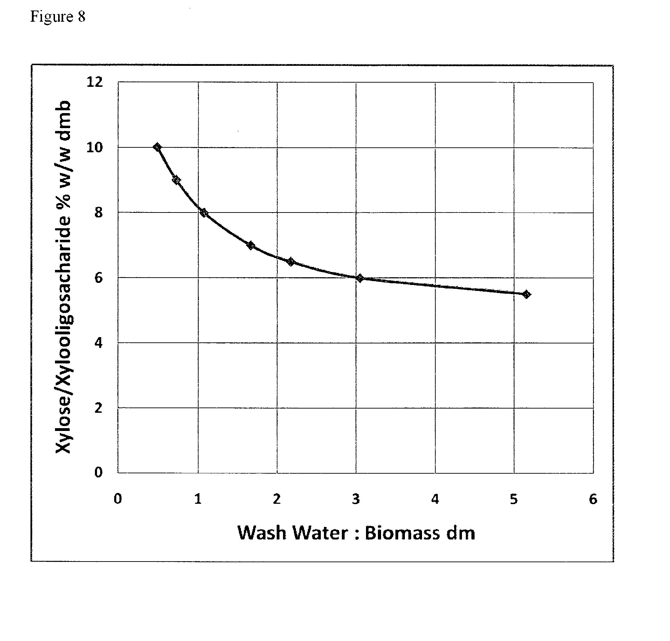

It should be noted that a more complex commercial system of washing could also be employed as described previously. The washing system could include multiple washers, presses, filters, or other equipment arranged with counter current and recycle streams to minimize the dilution factor while achieving the desired recovery of soluble impurities. A 2 stage counter current washing system, see figure 8, would gives a practical commercial ratio of about 3:1 (water:biomass) for a result of 6% xylose in the biomass solids.

### EXAMPLE

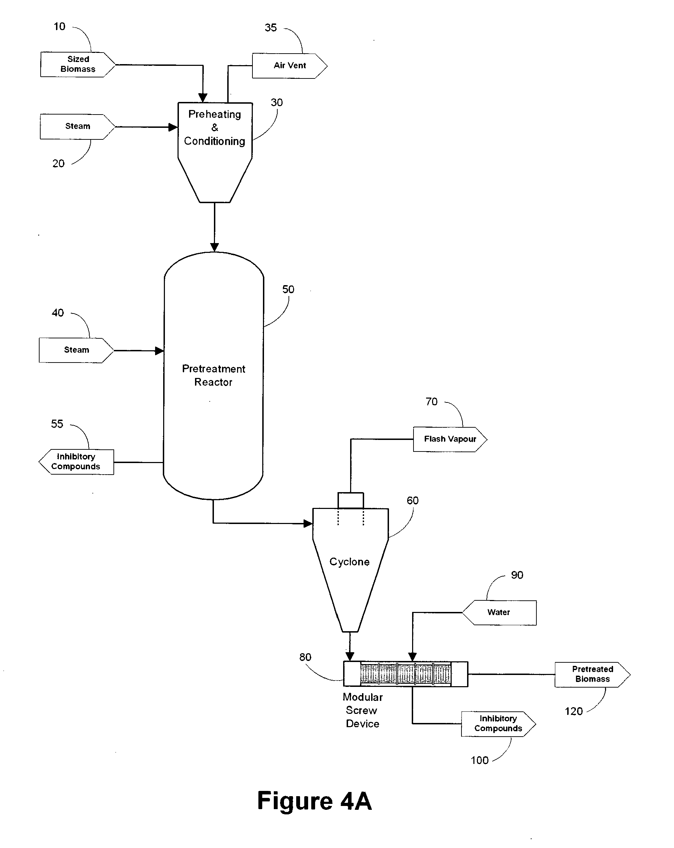

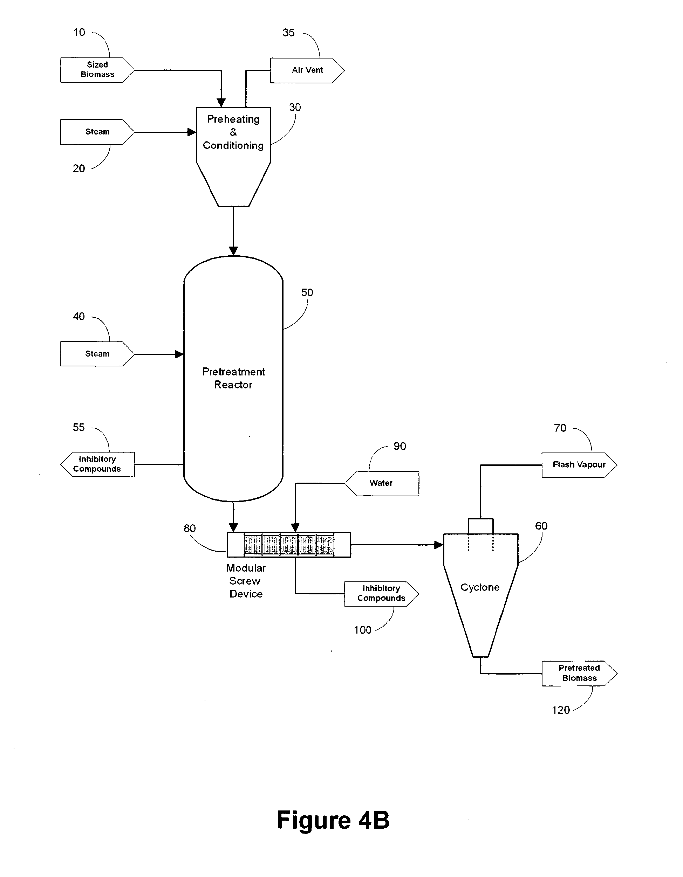

Batch steam explosion pretreatment of corncob was carried out in a steam gun (Figure 4A and 4B). The steam gun (50), was supplied with saturated steam from a steam storage vessel (40). Pre-steamed ground corncobs of 0.5 to 1 cm³ particle size were fed through a V shaped hopper and screw auger (from Genemco, not shown). The amount of each batch load was controlled by a weigh hopper. Batch loads of 6 kg corncob were used per steam explosion shot. Corncob weight and production rates are expressed on a dry matter basis. After filling the batch load into the steam gun (50) from above, a fill gate (not shown) was closed to seal the steam gun. Pressurized saturated steam until the desired cooking pressure was reached. Cooking pressures of 167 to 322 psig were used (12.6 to 23.2 bar). After a residence time of 3 to 10 minutes, at temperatures from 190 °C to 220 °C, the pressure in the steam gun was quickly released by opening a flash purge valve (not shown) located at the bottom of the steam gun. Complete pressure relief was achieved in up to 1000 ms. During the residence time and prior to pressure release, condensate and cooking liquids collected at the bottom of the steam gun were purged through a purge discharge control valve (55) and fed to a condensate collection system (not shown) through a purge conduit. Volatile reaction products generated during steam treatment were removed through the purge valve and directed to an environmental control unit (not shown) through a purge line. The solids collected at the bottom of the cyclone separator (60) were subjected to further processing in the lab. The gaseous components were collected and condensed (70) and fed to the condensate collection system. Any gaseous emissions from the steam gun, the cyclone separator and other parts of the setup were collected and treated in an environmental control unit (not shown). Cleaned gases were exhausted to atmosphere from the unit.

Pre-hydrolyzed cob dry matter was diluted 16:1 with fresh water (90). The slurry was pressed to 40 % solids in a hydraulic cylinder (80). The solids (120) were shredded in a garden shredder (not shown) and then diluted with fresh water to the desired consistency for hydrolysis and fermentation. The resulting xylose DM content achieved was 6% and the dilution factor was 6. Wash water containing hydrolyzed soluble hemicellulose products and toxic compounds, the inhibitory compounds (100), was collected and concentrated to the desired dryness for further applications.

Composition analysis of the wash water showed that over 80 % of the xylo-oligosaccharides present in the wet fraction of pretreated cob fibres were removed by water washing (Figure 2). Results of the 2.5 tonne pilot scale trial carried out showed that a concentration of 100 g/L glucose was reached at t_{90%} of 100 hours. An alcohol concentration of 5 % was reached in 20 hours.

The same process of washing of the pre-hydrolyzed dry matter was carried out at various different dilution ratios to determine the impact on downstream enzyme activity on the cellulose illustrated by the time (hrs) to 90% hydrolysis and the observed results are illustrated in Figures 1 and 6.

### Non-limiting CLAUSES

1. A process for pretreatment of lignocellulosic biomass, comprising the steps of heating the lignocellulosic biomass with steam to a preselected temperature, at a preselected pressure and for a preselected time to hydrolyze and solubilize hemicelluloses in the lignocellulosic biomass, explosively decomposing the lignocellulosic biomass by rapidly releasing the pressure to break down the lignocellulosic biomass into fibers and extracting from the resulting reaction mixture a liquefied portion of the lignocellulosic biomass before or after explosive decomposition, for removing compounds from the lignocellulosic biomass which are inhibitory to enzymatic cellulose hydrolysis and sugar fermentation to ethanol, wherein the extracting step is discontinued once a dry matter (dm) content of xylose, as monomer or oligomer, in the reaction mixture of 4% to 8% (w/w dm) is achieved.
2. The process of clause 1, wherein the xylose dm content is about 6%.
3. The process of clause 1, wherein the liquid extraction step is carried out by separating fibrous solids from the liquid of the pretreated lignocellulosic biomass.
4. The process of clause 3, wherein an eluent is used to increase the level of inhibitory extraction.
5. The process of clause 3, wherein the eluent extraction step is carried out under a pressure of up to 24 bar (350 psi).
6. The process of clause 5, wherein the liquid extraction step is carried out with a sealed mechanical compression device.
7. The process of clause 3, wherein the liquid extraction step is carried out under a pressure of up to 24 bar (350 psi) with a sealed mechanical compression device using an eluent to improve inhibitor removal.
8. The process of clause 4, wherein the eluent extraction step is carried out after pre-treatment with a screw press, a filter, a filter press, a belt press, a centrifuge or a drainer.
9. The process of clause 3, wherein the liquid extraction step is carried out after pretreatment with a screw press, a filter, a filter press, a belt press, a centrifuge or a drainer with the addition of an eluent.
10. The process of clause 3, wherein the liquid extraction step is carried during the pretreatment under pressure and after pretreatment after pressure reduction.
11. The process of clause 3, wherein the liquid extraction step is carried out during the pretreatment under pressure and after pretreatment with an eluent.
12. The process of clause 1 or 2, wherein the removal step is carried out for the removal of water soluble hydrolyzed hemicellulose and hemicellulose hydrolysis and degradation components and water soluble or suspended degradation products thereof.
13. The process of clause 12, wherein the water soluble hydrolyzed hemicellulose and hemicellulose hydrolysis and degradation products include xylo-oligosaccharides, xylose, mannose-, galactose-, rhamnose- and arabinose-based oligomer and monomer sugars, acetic acid and formic acid.
14. The process of clause 13, wherein other compounds inhibitory to downstream cellulose hydrolysis and fermentation processes are removed in the removing steps.
15. The process of clause 14 where the other compounds are fatty acids, sterols, esters, or ethers.
16. The process of clause 14, wherein soluble xylose oligomers created in the hemicellulose hydrolysis during pretreatment are 30% to 90% of the hydrolyzed xylan in the pretreated biomass.
17. The process of clause 1 or 2 , wherein the lignocellulosic biomass is pretreated by auto-hydrolysis or dilute acid catalysis.
18. The process of clause 1 or 2, wherein the removing is carried out with counter current washing using water as an eluent.
19. The process of clause 5 and 7 and 18, wherein eluent origins are from recycle streams or recycled eluent water.
20. The process of clause 1 or 2, wherein a combined xylose and xylo-oligosaccharide concentration in the pretreated biomass after the extracting/removing step is between 4% and 6% for minimizing an amount of cellulolytic enzymes required to hydrolyze the reactive cellulose obtained.

## Claims

1. A process for pretreatment of lignocellulosic biomass, comprising the steps of heating the lignocellulosic biomass with steam to a preselected temperature, at a preselected pressure and for a preselected time to hydrolyze and solubilize hemicelluloses in the lignocellulosic biomass, explosively decomposing the lignocellulosic biomass by rapidly releasing the pressure to break down the lignocellulosic biomass into fibers and extracting from the resulting reaction mixture a liquefied portion of the lignocellulosic biomass before or after explosive decomposition, for removing compounds from the lignocellulosic biomass which are inhibitory to enzymatic cellulose hydrolysis and sugar fermentation to ethanol, wherein the extracting step is discontinued once a dry matter (dm) content of xylose, as monomer or oligomer, in the reaction mixture of 4% to 8% (w/w dm) is achieved.

2. The process of claim 1, wherein the xylose dm content is about 6%.

3. The process of claim 1, wherein the liquid extraction step is carried out by separating fibrous solids from the liquid of the pretreated lignocellulosic biomass.

4. The process of claim 3, wherein an eluent is used to increase the level of inhibitory extraction, wherein optionally the eluent extraction step is carried out after pre-treatment with a screw press, a filter, a filter press, a belt press, a centrifuge or a drainer.

5. The process of claim 3, wherein the eluent extraction step is carried out under a pressure of up to 24 bar (350 psi), wherein optionally the liquid extraction step is carried out with a sealed mechanical compression device.

6. The process of claim 3, wherein the liquid extraction step is carried out under a pressure of up to 24 bar (350 psi) with a sealed mechanical compression device using an eluent to improve inhibitor removal.

7. The process of claim 3, wherein:
(1) the liquid extraction step is carried out after pretreatment with a screw press, a filter, a filter press, a belt press, a centrifuge or a drainer with the addition of an eluent;
(2) the liquid extraction step is carried out during the pretreatment under pressure and after pretreatment after pressure reduction; or
(3) the liquid extraction step is carried out during the pretreatment under pressure and after pretreatment with an eluent.

8. The process of claim 1 or 2, wherein the removal step is carried out for the removal of water soluble hydrolyzed hemicellulose and hemicellulose hydrolysis and degradation components and water soluble or suspended degradation products thereof.

9. The process of claim 8, wherein the water soluble hydrolyzed hemicellulose and hemicellulose hydrolysis and degradation products include xylo-oligosaccharides, xylose, mannose-, galactose-, rhamnose- and arabinose-based oligomer and monomer sugars, acetic acid and formic acid.

10. The process of claim 9, wherein other compounds inhibitory to downstream cellulose hydrolysis and fermentation processes are removed in the removing steps, wherein optionally the other compounds are fatty acids, sterols, esters, or ethers.

11. The process of claim 10, wherein soluble xylose oligomers created in the hemicellulose hydrolysis during pretreatment are 30% to 90% of the hydrolyzed xylan in the pretreated biomass.

12. The process of claim 1 or 2 , wherein the lignocellulosic biomass is pretreated by auto-hydrolysis or dilute acid catalysis.

13. The process of claim 1 or 2, wherein the removing is carried out with counter current washing using water as an eluent.

14. The process of claim 5 and 6 and 13, wherein eluent origins are from recycle streams or recycled eluent water.

15. The process of claim 1 or 2, wherein a combined xylose and xylo-oligosaccharide concentration in the pretreated biomass after the extracting/removing step is between 4% and 6% for minimizing an amount of cellulolytic enzymes required to hydrolyze the reactive cellulose obtained.

## Patentansprüche

1. Verfahren zur Vorbehandlung von lignozellulosehaltiger Biomasse umfassend die Schritte Erhitzen der lignozellulosehaltigen Biomasse mit Dampf auf eine vorgewählte Temperatur, bei einem vorgewählten Druck und für eine vorgewählte Zeit, um Hemizellulosen in der lignozellulosehaltigen Biomasse zu hydrolysieren und aufzulösen, explosionsartiges Zersetzen der lignozellulosehaltigen Biomasse durch schnelles Ablassen des Drucks, um die lignozellulosehaltige Biomasse in Fasern aufzuspalten und Extrahieren eines verflüssigten Teils der lignozellulosehaltigen Biomasse aus dem sich ergebenden Reaktionsgemisch vor oder nach der explosionsartigen Zersetzung, um Verbindungen aus der lignozellulosehaltigen Biomasse zu entfernen, die für eine enzymatische Zellulosehydrolyse und Zuckerfermentation zu Ethanol hemmend sind, wobei der Schritt des Extrahierens abgebrochen wird, sobald in dem Reaktionsgemisch ein Trockenmasse (dm)-Gehalt von Xylose, als Monomer oder Oligomer, von 4% bis 8% (Masseprozenz dm) erreicht wird.

2. Verfahren nach Anspruch 1, wobei der Xylose-dm-Gehalt etwa 6% ist.

3. Verfahren nach Anspruch 1, wobei der Flüssigkeitsextraktionsschritt durch ein Trennen fasriger Feststoffe von der Flüssigkeit der vorbehandelten lignozellulosehaltigen Biomasse vorgenommen wird.

4. Verfahren nach Anspruch 3, wobei ein Elutionsmittel verwendet wird, um den Grad der inhibitorischen Extraktion zu erhöhen, wobei wahlweise der Elutionsmittelextraktionsschritt nach einer Vorbehandlung mit einer Schneckenpresse, einem Filter, einer Filterpresse, einer Bandpresse, einer Zentrifuge oder einem Drainer vorgenommen wird.

5. Verfahren nach Anspruch 3, wobei der Elutionsmittelextraktionsschritt unter einem Druck von bis zu 24 bar (350 psi) vorgenommen wird, wobei wahlweise der Flüssigkeitsextraktionsschritt mit einer verschlossenen mechanischen Kompressionsvorrichtung vorgenommen wird.

6. Verfahren nach Anspruch 3, wobei der Flüssigkeitsextraktionsschritt unter einem Druck von bis zu 24 bar (350 psi) mit einer verschlossenen mechanischen Kompressionsvorrichtung vorgenommen wird, wobei ein Elutionsmittel verwendet wird, um die Entfernung von Inhibitor zu verbessern.

7. Verfahren nach Anspruch 3, wobei
(1) der Flüssigkeitsextraktionsschritt nach einer Vorbehandlung mit einer Schneckenpresse, einem Filter, einer Filterpresse, einer Bandpresse, einer Zentrifuge oder einem Drainer mit dem Zusatz eines Elutionsmittels vorgenommen wird;
(2) der Flüssigkeitsextraktionsschritt während der Vorbehandlung unter Druck und nach der Vorbehandlung nach Drucksenkung vorgenommen wird; oder
(3) der Flüssigkeitsextraktionsschritt während der Vorbehandlung unter Druck und nach der Vorbehandlung mit einem Elutionsmittel vorgenommen wird.

8. Verfahren nach Anspruch 1 oder 2, wobei der Entfernungsschritt zur Entfernung von wasserlöslicher hydrolysierter Hemizellulose und Hydrolyse- und Abbaubestandteilen von Hemizellulose und wasserlöslichen oder suspendierten Abbauprodukten davon vorgenommen wird.

9. Verfahren nach Anspruch 8, wobei die wasserlösliche hydrolysierte Hemizellulose und Hydrolyse- und Abbauprodukte von Hemizellulose Xylooligosaccharide, Xylose, Mannose-, Galaktose-, Rhamnose- und Arabinosebasierte Oligomer- und Monomerzucker, Essigsäure und Ameisensäure einschließen.

10. Verfahren nach Anspruch 9, wobei andere Verbindungen, die für die nachgeschalteten Zellulosehydrolyse- und Fermentationsverfahren hemmend sind, in den Entfernungsschritten entfernt werden, wobei wahlweise die anderen Verbindungen Fettsäuren, Sterine, Ester oder Ether sind.

11. Verfahren nach Anspruch 10, wobei lösliche Xyloseoligomere, die in der Hydrolyse von Hemizellulose während der Vorbehandlung entstanden sind, 30% bis 90% des hydrolysierten Xylans in der vorbehandelten Biomasse ausmachen.

12. Verfahren nach Anspruch 1 oder 2, wobei die lignozellulosehaltige Biomasse durch Autohydrolyse oder verdünnte Säure-Katalyse vorbehandelt wird.

13. Verfahren nach Anspruch 1 oder 2, wobei das Entfernen mit Gegenstromwaschen unter Verwendung von Wasser als ein Elutionsmittel vorgenommen wird.

14. Verfahren nach Anspruch 5 und 6 und 13, wobei das Elutionsmittel von Recyclingströmen oder recyceltem Elutionsmittelwasser stammt.

15. Verfahren nach Anspruch 1 oder 2, wobei eine kombinierte Xylose- und Xylooligosaccharidkonzentration in der vorbehandelten Biomasse nach dem Extrahieren-/Entfernen-Schritt zwischen 4% und 6% beträgt, um eine Menge von Zellulose abbauenden Enzymen, die benötigt werden, um die gewonnene reaktive Zellulose zu hydrolysieren, zu minimieren.

## Revendications

1. Procédé de prétraitement de biomasse lignocellulosique comprenant les étapes consistant à chauffer la biomasse lignocellulosique à la vapeur jusqu'à une température présélectionnée, à une pression présélectionnée et pendant un temps présélectionné pour hydrolyser et solubiliser les hémicelluloses dans la biomasse lignocellulosique, à décomposer de façon explosive la biomasse lignocellulosique en libérant rapidement la pression pour décomposer la biomasse lignocellulosique en fibres et à extraire du mélange de réaction résultant une portion liquéfiée de la biomasse lignocellulosique avant ou après la décomposition explosive pour éliminer de la biomasse lignocellulosique des composés qui sont inhibiteurs de l'hydrolyse enzymatique de la cellulose et la fermentation de sucre pour produire de l'éthanol, procédé, dans lequel l'étape d'extraction est interrompue une fois un contenu de matière sèche (ms) de 4% à 8% (m/m ms) de xylose sous forme de monomère ou d'oligomère est atteint dans le mélange de réaction.

2. Procédé suivant la revendication 1, dans lequel la teneur ms de xylose est de 6% environ.

3. Procédé suivant la revendication 1, dans lequel l'étape d'extraction est exécutée en séparant des solides fibreux du liquide de la biomasse lignocellulosique prétraitée.

4. Procédé suivant la revendication 3, dans lequel un éluant est utilisé pour augmenter le niveau d'extraction inhibitrice, dans lequel l'étape d'extraction à l'aide de l'éluant est optionnellement exécutée après prétraitement avec une presse à vis, un filtre, un filtre-presse, une presse à bande, une centrifugeuse ou un égouttoir.

5. Procédé suivant la revendication 3, dans lequel l'étape d'extraction à l'aide de l'éluant est exécutée sous une pression jusqu'à 24 bars (350 psi), l'étape d'extraction liquide étant exécutée optionnellement avec un dispositif de compression mécanique étanchéifié.

6. Procédé suivant la revendication 3, dans lequel l'étape d'extraction liquide est exécutée sous une pression jusqu'à 24 bars (350 psi) avec un dispositif de compression mécanique étanchéifié en utilisant un éluant pour améliorer l'élimination d'inhibiteurs.

7. Procédé suivant la revendication 3, dans lequel:
(1) l'étape d'extraction liquide est exécutée après prétraitement avec une presse à vis, un filtre, un presse-filtre, une presse à bande, une centrifugeuse ou un égouttoir avec addition d'un éluent;
(2) l'étape d'extraction liquide est exécutée durant le prétraitement sous pression et après celui-ci, après libération de la pression, ou
(3) l'étape d'extraction liquide est exécutée durant le prétraitement sous pression et après prétraitement avec un éluant.

8. Procédé suivant la revendication 1 ou 2, dans lequel l'étape d'élimination est exécutée pour éliminer l'hémicellulose hydrolysée soluble dans l'eau et les composés d'hydrolyse de l'hémicellulose et de dégradation et les produits de dégradation solubles ou en suspension dans l'eau de ceux-ci.

9. Procédé suivant la revendication 8, dans lequel l'hémicellulose hydrolysée soluble dans l'eau et les produits d'hydrolyse de l'hémicellulose et de dégradation comprennent des xylo-oligosaccharides, du xylose, des oligomères à base de mannose, galactose, rhamnose et arabinose, ainsi que de l'acide acétique et de l'acide formique.

10. Procédé suivant la revendication 9, dans lequel d'autres composants inhibiteurs d'hydrolyse de cellulose et de processus de fermentation en aval sont éliminés au cours des étapes d'élimination, les autres composants étant optionnellement des acides gras, stérols, esters ou éthers.

11. Procédé suivant la revendication 10, dans lequel des oligomères de xylose solubles créés par l'hydrolyse d'hémicellulose au cours du prétraitement constituent entre 30% et 90% du xylane hydrolysé dans la biomasse prétraitée.

12. Procédé suivant la revendication 1 ou 2, dans lequel la biomasse lignocellulosique est prétraitée par auto-hydrolyse ou catalyse d'acide dilué.

13. Procédé suivant la revendication 1 ou 2, dans lequel l'élimination est exécutée à l'aide d'un lavage à contre-courant utilisant de l'eau comme éluant.

14. Procédé suivant les revendications 5 et 6 et 13, dans lequel l'éluant provient de courants de recyclage ou d'eau d'élution recyclée.

15. Procédé suivant la revendication 1 ou 2, dans lequel une concentration combinée de xylose et de xylo-oligosaccharides dans la biomasse prétraitée après l'étape d'extraction/d'élimination est comprise entre 4% et 6% pour minimiser une quantité d'enzymes cellulolytiques requise pour hydrolyser la cellulose réactive obtenue.
